# EUROPEAN PATENT APPLICATION

(11) **EP 2 425 830 A1**
(43) Date of publication of application: **07.03.2012**
(21) Application number: 10075382.1
(22) Date of filing: 03.09.2010
(51) Int. Cl.: A61K 31/131, A61P 35/00

(54) **Synergistic drug combination for the treatment of cancer**

(71) Applicant: Max-Planck-Gesellschaft zur Förderung der Wissenschaften e.V., 80539 München (DE)
(72) Inventor: Gusenbauer, Simone, 82152 Martinsried (DE); Ullrich, Axel, 80331 Munich (DE)
(74) Representative: Arth, Hans-Lothar

(57) **Abstract**

The present invention relates to synergistic drug combinations and the use of said drug combinations for the treatment of proliferative diseases.

## Description

The present invention relates to synergistic drug combinations and the use of said drug combinations for the treatment of proliferative diseases.

The concept of combining differing agents to treat a person in need of such treatment has already been suggested several times in the art.

US 20090263397 for example discloses a method for treating tumors or tumor metastases in a patient, comprising administering to said patient simultaneously or sequentially a therapeutically effective amount of a combination of an anti-cancer agent or treatment that elevates pAkt levels in tumor cells and an IGF-1 R kinase inhibitor.

US 20090221615 discloses a method of treating cancer in a mammal comprising administering Lucanthone to the mammal and administering at least one antimetabolite to the mammal.

US 7510830 provides a method and a composition for treating cancer, the method including the step of administering, either sequentially or simultaneously an agent of the xanthenone acetic acid group of compounds, and another agent selected from agents which modulate TNF production and compounds which act on biochemical pathways leading to TNF synthesis.

Even if the concept of combining several pharmaceutical agents has already been suggested, and although various combinations are under investigation and in clinical trials, there is still a need for new and efficient pharmaceutical agents for the treatment of diseases in which cell proliferation, migration or apoptosis of myeloma cells, or angiogenesis are involved, and there is still a need to develop further combinations which can show increased efficacy and reduced side-effects.

It is object of the present invention to provide further drug combinations for the treatment of proliferative diseases such as cancer, tumors, cancer metastases and particularly drug resistant tumors and cancers.

The objective of the present invention is solved by the teaching of the independent claims. Further advantageous features, aspects and details of the invention are evident from the dependent claims, the description, and the examples of the present application.

Surprisingly it has been found that the inventive drug combination comprising a pharmaceutically active agent A and a supporting agent B or synergistic agent B lead to a pharmaceutical composition which is more effective than the single agents A or B. The inventive drug combination is useful to treat proliferative diseases more efficiently and is especially usful to treat drug resistant proliferative diseases and more especially to treat drug resistant proliferative diseases wherein the proliferative disease is resistant to agent A or agent B and especially is resistant to agent B.

Consequently the present invention relates to the surprising finding that a combination of agent A and agent B is more effective in cancer treatment than the agent A alone or agent B alone. Moreover cancer types or cancers which developed resistance to agent A were sensitive to the treatment with the drug combiantion of agent A and agent B and on the other hand, cancer types or cancers which developed resistance to agent B were sensitive to the treatment with the drug combination of agent B and agent A. Therefore the second agent of the drug combination can be regarded as supporting agent for the first agent of the drug combination. This means, if a proliferative disease was treated with agent A treatment efficacy can be increased or a drug resistance to agent A can be overcome by administration of a combination of agent A together with agent B. However the other way round is more preferred which means that if a proliferative disease was treated with agent B treatment efficacy can be increased or a drug resistance to agent B can be overcome by administration of a combination of agent B together with agent A. Until now, no development of resistance to the drug combination of agent A and agent B could be observed.

According to the invention it has been found that co-administration to a person in need of such treatment with effective amounts of a pharmaceutically active agent A and a pharmaceutically active agent B is beneficial to the person in need of such treatment and is mainly based on the additive and synergistic effects of the combined treatment, or to an improved tolerability of the treatment by the patient due to, for example, the administration of lower doses of the pharmaceutical agents involved.

Further it has been found that the diseases which can be treated by the combination of the pharmaceutical agents in accordance with the present invention are all kind of diseases in which cell proliferation, migration or apoptosis of myeloma cells, or angiogenesis are involved, which can be of oncological nature such as all types of malignant neoplasias or cancers.

This invention relates to a method for the treatment of diseases involving cell proliferation, migration or apoptosis of myeloma cells, or angiogenesis, which method comprises administering either sequentially or simultaneously to a person in need of such treatment with effective amounts of:
(i) at least one pharmaceutically active agent A, selected from antiproliferative agents and
(ii) at least one pharmaceutically active agent B, selected from supporting agents able to increase the antiproliferative effect of agent A and/or able to decrease drug resistance to agent A
or
(i*) at least one pharmaceutically active agent B, selected from antiproliferative agents and
(ii*) at least one pharmaceutically active agent A, selected from supporting agents able to increase the antiproliferative effect of agent B and/or able to decrease drug resistance to agent B.

The pharmaceutically active agent A can be selected from the list comprising or consisting of: chlorethamine, cyclophosphamide (cytoxan), trofosfamide, ifosfamide (Ifex), melphalan (Alkeran), mechlorethamine hydrochloride (Mustargen), chlorambucil (Leukeran), busulfan (Myleran), thiotepa, 2-cyano-3-(3,4-dihydroxyphenyl)-N-(phenylmethyl)-2-propenamide, carmustine, carboplalomustine, dacarbazine, procarbazine, erlotinib, temozolomide, treosulfan, estramustine, N-(3-(5-chloro-2-(2-methoxy-4-(4-methylpiperazin-1-yl)phenylamino)pyrimidin-4-yloxy)phenyl)acrylamide, nimustine, methotrexate (MTX, Mexate), 5-fluorouracil (Fluoracil, 5-FU), 6-thioguanine (Thioguanine, 6-TG), 6-mercaptopurine, (N-[-4-[(3-Chloro-4-fluorophenyl)amino]-7-[3-(4-morpholinyl)propoxy]-6-quinazolinyl]-2-propenamide), N-(3-chlorophenyl)-6,7-dimethoxy-4-quinazolinamine, fludarabine (Fludara), floxuridine (FUDR), cladribine, pentostatin, gemcitabine (Gemzar), cytarabine, azathioprine, raltitrexed, capecitabine (Xeloda), 4-phenethylamino-6-(yderoxyl)phenyl-7H-pyrrolo(2,3-d)pyrimidine, cytosine arabinoside, deoxycoformycin (Pentostatin, Nipent), lapatinib, thioguanine, 7-(4-(3-ethynylphenylamino)-7-methoxyquinazolin-6-yloxy)-N-hydroxyheptanamide, mercaptopurine (6-MP, Purinethol), paclitaxel, docetaxel, hydroxycarbamide (hydroxyurea), imatinibe, Miltefosine^{®}, N-(3-(5-chloro-2-(4-(4-methylpiperazin-1-yl)phenylamino)pyrimidin-4-yloxy)phenyl)acrylamide, cetuximab, amsacrine, 4-[(3-Bromophenyl)amino]-6,7-dimethoxyquinazoline hydrochloride), pentostatin, bexarotene, tretinoin, asparaginase, trastuzumab (also known as Herceptin^{®}), alemtuzumab (also known as MabCampath^{®}), rituximab (also known as MabThera^{®}), N-[4-[(3-Chloro-4-fluorophenyl)amino]-7-[[(3"S")-tetrahydro-3-furanyl]oxy]-6-quinazolinyl]-4(dimethylamino)-2-butenamide, glucocorticoids (prednisone), hydrocortisones, gefitinib, dexamethasones (Decadron), (E)-N-(4-((3-chloro-4-fluorophenyl)amino)-3-cyano-7-ethoxyquinolin-6-yl)-4-(dimethylamino)but-2-enamide, estrogens [fosfestrol, estramustine (Emcyt), chlorotrianisene (TACE), diethylstilbestrol (DES)], (R)-6-(4-((4-methylpiperazin-1-yl)methyl)phenyl)-N-(1-phenylethyl)-7H-pyrrolo[2,3-d]pyrimidin-4-amine, aromatase inhibitors [anastrozole (Arimidex)], LHRH (buserelin, goserelin, leuprorelin, triptorelin), N-(3-Chloro-4-((3-fluorophenyl)methoxy)phenyl)-6-(5-(((2-(methylsulfonyl)ethyl)amino)methyl)-2-furanyl)-4-quinazolinamine bis(4-methylbenzenesulfonate), flutamide (Eulexing), bicalutamide (Casodex), leuprolide (Lupron), goserelin acetate (Zoladex), [4-[[1-(3-fluorophenyl)methyl]-1H-indazol-5-ylamino]-5-methylpyrrolo[2,1-f][1,2,4]triazin-6-yl]carbamic Acid (3S)-3-Morpholinylmethyl Ester, vandetanib, cyproterone acetate, N-(3-(5-chloro-2-(4-(4-methylpiperazin-1-yl)phenylamino)pyrimidin-4-ylthio)phenyl)acrylamide, panitumumab, progestine (Medoxyprogesterone acetate [(Depo-provera)], megestrol acetate (Megacel), tamoxifen and toremifen.

Where no generic name or chemical nomenclature was available the code name of the pharmaceutically active agent has been listed. Behind each code name the name of the developing company or alternatively the name of the manufacturing company has been added in parentheses. The names of companies are always further characterized by the addition of the abbreviation Inc.

The agent "Amix" consists of three mouse anti-human HGF/SF monoclonal antibodies (A-1, A-7 and A-10) and is described in the publication Cao et al., PNAS, June 19, 2001, 98(13); 7443-48 and were produced according to material & methods on pages 7443 and 7444 and as shown in table 1 of said publication.
The antibodies "1.29.1, 1.74.1, 1.75.1, 2.4.4 and 2.12.1" are described in the publication Burgess et al., Cancer Res, February 1, 2006, 66; 1721 and were produced according to material & methods on pages 1722 - 1725 and as shown in table 1 of said publication.

More preferably, the pharmaceutically active agent A can be selected from the list comprising, cyclophosphamide (cytoxan), trofosfamide, ifosfamide (Ifex), mechlorethamine hydrochloride (Mustargen), chlorambucil (Leukeran), busulfan (Myleran), thiotepa, 2-cyano-3-(3,4-dihydroxyphenyl)-N-(phenylmethyl)-2-propenamide, carboplalomustine, dacarbazine, procarbazine, erlotinib, temozolomide, treosulfan, N-(3-(5-chloro-2-(2-methoxy-4-(4-methylpiperazin-1-yl)phenylamino)pyrimidin-4-yloxy)phenyl)acrylamide, nimustine, methotrexate (MTX, Mexate), 6-thioguanine (Thioguanine, 6-TG), 6-mercaptopurine, N-[-4-[(3-Chloro-4-fluorophenyl)amino]-7-[3-(4-morpholinyl)propoxy]-6-quinazolinyl]-2-propenamide, N-(3-chlorophenyl)-6,7-dimethoxy-4-quinazolinamine, fludarabine (Fludara), floxuridine (FUDR), cladribine, pentostatin, gemcitabine (Gemzar), cytarabine, capecitabine (Xeloda), 4-phenethylamino-6-(yderoxyl)phenyl-7H-pyrrolo(2,3-d)pyrimidine, cytosine arabinoside, deoxycoformycin (Pentostatin, Nipent), lapatinib, thioguanine, 7-(4-(3-ethynylphenylamino)-7-methoxyquinazolin-6-yloxy)-N-hydroxyheptanamide, mercaptopurine (6-MP, Purinethol), paclitaxel, docetaxel, hydroxycarbamide (hydroxyurea), imatinibe, Miltefosine^{®}, N-(3-(5-chloro-2-(4-(4-methylpiperazin-1-yl)phenylamino)py- rimidin-4-yloxy)phenyl)acrylamide, cetuximab, amsacrine, 4-[(3-Bromophenyl)amino]-6,7-dimethoxyquinazoline hydrochloride, bexarotene, tretinoin, trastuzumab (also known as Herceptin^{®}), alemtuzumab (also known as MabCampath^{®}), rituximab (also known as MabThera^{®}), N-[4-[(3-Chloro-4-fluorophenyl)amino]-7-[[(3"S")-tetrahydro-3-furanyl]oxy]-6-quinazolinyl]-4(dimethyl amino)-2-butenamide, gefitinib, dexamethasones (Decadron), (E)-N-(4-((3-chloro-4-fluorophenyl)amino)-3-cyano-7-ethoxyquinolin-6-yl)-4-(dimethylamino)but-2-enamide, (R)-6-(4-((4-methylpiperazin-1-yl)methyl)phenyl)-N-(1-phenylethyl)-7H-pyrrolo[2,3-d]pyrimidin-4-amine, estramustine (Emcyt), chlorotrianisene (TACE), diethylstilbestrol (DES)], LHRH (buserelin, goserelin, leuprorelin, triptorelin), N-(3-Chloro-4-((3-fluorophenyl)methoxy)phenyl)-6-(5-(((2-(methylsulfonyl)ethyl)amino) methyl)-2-furanyl)-4-quinazolinamine bis(4-methylbenzenesulfonate), flutamide (Eulexing), bicalutamide (Casodex), leuprolide (Lupron), goserelin acetate (Zoladex), [4-[[1-(3-fluorophenyl)methyl]-1H-indazol-5-ylamino]-5-methylpyrrolo[2,1-f][1,2,4]triazin-6-yl]carbamic Acid (3S)-3-Morpholinylmethyl Ester, N-(3-(5-chloro-2-(4-(4-methylpiperazin-1-yl)phenylamino)pyrimidin-4-ylthio)phenyl)acryl-amide), panitumumab, progestine (Medoxyprogesterone acetate [(Depoprovera)] and megestrol acetate (Megacel).

Even more preferred, the pharmaceutically active agent A can be selected from cyclophosphamide (cytoxan), mechlorethamine hydrochloride (Mustargen), chlorambucil (Leukeran), 2-cyano-3-(3,4-dihydroxyphenyl)-N-(phenylmethyl)-2-propenamide, carboplalomustine, erlotinib, temozolomide, treosulfan, N-(3-(5-chloro-2-(2-methoxy-4-(4-methylpiperazin-1-yl)phenylamino)pyrimidin-4-yloxy)phenyl)acrylamide, methotrexate (MTX, Mexate), 6-thioguanine (Thioguanine, 6-TG), 6-mercaptopurine, N-[-4-[(3-Chloro-4-fluorophenyl)amino]-7-[3-(4-morpholinyl)propoxy]-6-quinazolinyl]-2-propenamide, N-(3-chlorophenyl)-6,7-dimethoxy-4-quinazolinamine, fludarabine (Fludara), floxuridine (FUDR), gemcitabine (Gemzar), cytarabine, capecitabine (Xeloda), 4-phenethylamino-6-(yderoxyl)phenyl-7H-pyrrolo(2,3-d)pyrimidine, lapatinib, thioguanine, 7-(4-(3-ethynylphenylamino)-7-methoxyquinazolin-6-yloxy)-N-hydroxyheptan-amide, mercaptopurine (6-MP, Purinethol), paclitaxel, docetaxel, hydroxycarbamide (hydroxyurea), imatinibe, Miltefosine^{®}, N-(3-(5-chloro-2-(4-(4-methylpiperazin-1-yl)phenylamino)pyrimidin-4-yloxy)phenyl)acrylamide, cetuximab, amsacrine, 4-[(3-Bromophenyl)amino]-6,7-dimethoxyquinazoline hydrochloride, trastuzumab (also known as Herceptin^{®}), alemtuzumab (also known as MabCampath^{®}), rituximab (also known as MabThera^{®}), N-[4-[(3-Chloro-4-fluorophenyl)amino]-7-[[(3"S")-tetrahydro-3-furanyl] oxy]-6-quinazolinyl]-4(dimethylamino)-2-butenamide, gefitinib, (E)-N-(4-((3-chloro-4-fluorophenyl)amino)-3-cyano-7-ethoxyquinolin-6-yl)-4-(dimethylamino)but-2-enamide, (R)-6-(4-((4-methylpiperazin-1-yl)methyl)phenyl)-N-(1-phenylethyl)-7H-pyrrolo[2,3-d]pyrimidin-4-amine, estramustine (Emcyt), chlorotrianisene (TACE), diethylstilbestrol (DES)], LHRH (buserelin, goserelin, leuprorelin, triptorelin), N-(3-Chloro-4-((3-fluorophenyl)methoxy)phenyl)-6-(5-(((2-(methylsulfonyl)ethyl)amino)methyl)-2-furanyl)-4-quinazolinamine bis(4-methylbenzenesulfonate), leuprolide (Lupron), goserelin acetate (Zoladex), [4-[[1-(3-fluorophenyl)methyl]-1H-indazol-5-ylamino]-5-methylpyrrolo[2,1-f][1,2,4]triazin-6-yl]carbamic Acid (3S)-3-Morpholinylmethylester, N-(3-(5-chloro-2-(4-(4-methylpiperazin-1-yl)phenylamino)pyrimidin-4-ylthio)phenyl)acryl amide, panitumumab.

The pharmaceutically active agent B can be selected from the list comprising, daunorubicin (Daunomycin, Cerubidine), doxorubicin (adriamycin), liposomal adriamycin, dactinomycin, mitomycin C, (R,Z)-5-((2,6-dichlorobenzyl)sulfonyl)-3-((3,5-dimethyl-4-(2-(pyrrolidin-1-ylmethyl)pyrrolidine-1-carbonyl)-1 H-pyrrol-2-yl)methylene) indolin-2-one, bleomycin (Blenoxane), epirubicin (4-epi-adriamycin), idarubicin (Idamycin), AMG102 (Amgen, Inc.), dactinomycin (Actinomycin D, Cosmegen), mitoxantrone (Novantrone), AMG-208 (Amgen, Inc.), RP1040 (Incozen, Inc.), CEP-A (Cephalon, Inc.), N-[2-Fluoro-4-({2-[4-(4-methylpiperazin-1-yl)piperidin-1-yl]carbonylaminopyridin-4-yl}oxy)phenyl]-N'-(4-fluorophenyl)cyclopropane-1,1-dicarboxamide(2R,3R)-tartrate, MK8033 (Merck, Inc.), BMS777607 (Bristol-Myers Squibb, Inc.), GSK1363089/GSK089 (GlaxoSmithKline, Inc.), mitomycin C (Mitomycin), plicamycin (Mithracin), amsacrine, actinomycin D, vincristine, vinblastine, SCH900105 (AV-299) (Aveo Pharmaceuticals, Inc.), vindesine, etoposide, XL184 (Exelixis, Inc.), N-((2R)-1,4-Dioxan-2-ylmethyl)-N-methyl-N'-[3-(1-methyl-1 H-pyrazol-4-yl)-5-oxo-5H-benzo[4,5]cyclohepta[1,2-b]pyridin-7-yl]sulfamide, PF-04254644 (Pfizer, Inc.), N-(1,3-Benzodioxol-5-ylmethyl)-4-benzofuro[3,2-D]pyrimidin-4-yl-1-piperazinecarbothioamide, teniposide, cisplatin, carboplatin, 9S-(9a,10β,12α))-2,3,9,10,11,12-hexahydro-10-hydroxy-10-(methoxycarbonyl)-9-methyl-9,12-epoxy-1H-diindolo[1,2,3-fg:3',2',1'-kl]pyrrolo[3,4-i][1,6]benzodiazocin-1-one, oxaliplatin, vinorelbine, etoposide (VP-16, Etopophos, VePesid), ARQ197 (ArQule, Inc.), teniposide (VM-26, Vumon) topotecan (Hycamtin), irinotecan (Camptosar), carmustine (BCNU), lomustine (CCNU), TAK-701 (Millenium/Taked, Inc.), Amix, ab1.29.1, ab1.74.1, ab1.75.1, ab2.4.4, ab2.12.1, HGF splice variants NK2 and NK4, uncleaveable HGF, CGEN241 (Compugen, Inc.), OA-5D5 (Genentech, Inc.), isolated c-met Sema domain, DN30 (Metheresis Translational Res., Inc.), L2G7 (Galaxy Biotech, Inc.), PF-02341066 (Pfizer, Inc.), JNJ-38877605 (Johnson & Johnson, Inc.), streptozocin (Zanosar), 1,3-Dihydro-3-[(3,5-dimethyl-1 H-pyrrol-2-yl)methylene]-2H-indol-2-one, camptothecin, topotecan, SGX-523 (SGX Pharmaceuticals, Inc.), irinotecan, aminoglutethimide, formestane, (3Z)-N-(3-Chlorophenyl)-3-({3,5-dimethyl-4-[(4-methylpiperazin-1-yl)carbonyl]-1H-pyrrol-2-yl}methylene)-N-methyl-2-oxo-2,3-dihydro-1H-indole-5-sulfonamide, XL880 (Exelixis, Inc.), exemestane, letrozole, anastrozole, MGCD-265 (MethylGene, Inc.), interleukin-2, interferon-α, genestein, erythropoietin, G-CSF, ibritumomab (Zevalin^{®}), levamisole, asparaginase (Elspar), pegaspargase (Oncaspar), hydroxyurea (Hydrea, Mylocel), 2-(4-(1-(quinolin-6-ylmethyl)-1H-[1,2,3]triazolo[4,5-b]pyrazin-6-yl)-1H-pyrazol-1-yl)ethanol, procarbazine (Matulane) and imatinib mesylate (Gleevec), Neovastat, bevamizumab, avastin, angiozyme, 3-[(1R)-1-(2,6-dichloro-3-fluorophenyl)ethoxy]-5-(1-piperidin-4-ylpyrazol-4-yl)pyridin-2-amine, endostatin, angiostatin, combrestatin, vitaxin, geldanamycin carboxyamido thiazole, celecoxib and tirapazamine.

More preferably, the pharmaceutically active agent B can be selected from the list comprising, doxorubicin (adriamycin), liposomal adriamycin, mitomycin C, (R,Z)-5-((2,6-dichlorobenzyl)sulfonyl)-3-((3,5-dimethyl-4-(2-(pyrrolidin-1-ylmethyl)pyrrolidine-1-carbonyl)-1H-pyrrol-2-yl)methylene)indolin-2-one, bleomycin (Blenoxane), epirubicin (4-epi-adriamycin), idarubicin (Idamycin), AMG102 (Amgen, Inc.), dactinomycin (Actinomycin D, Cosmegen), mitoxantrone (Novantrone), AMG-208 (Amgen, Inc.), plicamycin (Mithracin), amsacrine, actinomycin D, vincristine, SCH900105 (AV-299) (Aveo Pharmaceuticals, Inc.), RP1040 (Incozen, Inc.), CEP-A (Cephalon, Inc.), N-[2-Fluoro-4-({2-[4-(4-methylpiperazin-1-yl)piperidin-1-yl]carbonylaminopyridin-4-yl}oxy)phenyl]-N'-(4-fluorophenyl)cyclopropane-1,1-dicarboxamide(2R,3R)-tartrate, MK8033 (Merck, Inc.), BMS777607 (Bristol-Myers Squibb, Inc.), GSK1363089/GSK089 (GlaxoSmithKline, Inc.), vindesine, etoposide, XL184 (Exelixis, Inc.), N-((2R)-1,4-Dioxan-2-ylmethyl)-N-methyl-N'-[3-(1-methyl-1H-pyrazol-4-yl)-5-oxo-5H-benzo-[4,5]cyclo-hepta[1,2-b]pyridin-7-yl]sulfamide, PF-04254644 (Pfizer, Inc.), N-(1,3-Benzodioxol-5-ylmethyl)-4-benzofuro[3,2-D]pyrimidin-4-yl-1-piperazinecarbothioamide, teniposide, carboplatin, 9S-(9α,10β,12α))-2,3,9,10,11,12-hexahydro-10-hydroxy-10-(methoxycarbonyl)-9-methyl-9,12-epoxy-1H-diindolo[1,2,3-fg:3',2',1'-kl]pyrrolo[3,4-i]-[1,6]benzodiazocin-1-one, oxaliplatin, vinorelbine, etoposide (VP-16, Etopophos, VePesid), ARQ197 (ArQule, Inc.), teniposide (VM-26, Vumon) topotecan (Hycamtin), irinotecan (Camptosar), TAK-701 (Millenium/Taked, Inc.), Amix, ab1.29.1, ab1.74.1, ab1.75.1, ab2.4.4, ab2.12.1, HGF splice variants NK2 and NK4, uncleaveable HGF, CGEN241 (Compugen, Inc.), OA-5D5 (Genentech, Inc.), isolated c-met Sema domain, DN30 (Metheresis Translational Res., Inc.), L2G7 (Galaxy Biotech, Inc.), PF-02341066 (Pfizer, Inc.), carmustine (BCNU), lomustine (CCNU), JNJ-38877605 (Johnson & Johnson, Inc.), streptozocin (Zanosar), 1,3-Dihydro-3-[(3,5-dimethyl-1H-pyrrol-2-yl)methylene]-2H-indol-2-one, camptothecin, SGX-523 (SGX Pharmaceuticals, Inc.), irinotecan. aminoglutethimide, formestane, (3Z)-N-(3-Chlorophenyl)-3-({3,5-dimethyl-4-[(4-methylpiperazin-1-yl)carbonyl]-1H-pyrrol-2-yl}methylene)-N-methyl-2-oxo-2,3-dihydro-1 H-indole-5-sulfonamide, XL880 (Exelixis, Inc.), exemestane, letrozole, anastrozole, MGCD-265 (MethylGene, Inc.), genestein, erythropoietin, G-CSF, ibritumomab (Zevalin^{®}), levamisole, asparaginase (Elspar), pegaspargase (Oncaspar), hydroxyurea (Hydrea, Mylocel), 2-(4-(1-(quinolin-6-ylmethyl)-1H-[1,2,3]triazolo[4,5-b]pyrazin-6-yl)-1H-pyrazol-1-yl)ethanol, procarbazine (Matulane) and imatinib mesylate (Gleevec), bevamizumab, avastin, angiozyme, 3-[(1R)-1-(2,6-dichloro-3-fluorophenyl)ethoxy]-5-(1-piperidin-4-ylpyrazol-4-yl)pyridin-2-amine, endostatin, angiostatin, vitaxin, geldanamycin carboxyamido thiazole and celecoxib.

Even more preferred, the pharmaceutically active agent B can be selected from (R,Z)-5-((2,6-dichlorobenzyl)sulfonyl)-3-((3,5-dimethyl-4-(2-(pyrrolidin-1-ylmethyl) pyrrolidine-1-carbonyl)-1H-pyrrol-2-yl)methylene)indolin-2-one, bleomycin (Blenoxane), epirubicin (4-epi-adriamycin), idarubicin (Idamycin), AMG102 (Amgen, Inc.), dactinomycin (Actinomycin D, Cosmegen), mitoxantrone (Novantrone), AMG-208 (Amgen, Inc.), plicamycin (Mithracin), vincristine, SCH900105 (AV-299) (Aveo Pharmaceuticals, Inc.), vindesine, XL184 (Exelixis, Inc.), RP1040 (Incozen, Inc.), CEP-A (Cephalon, Inc.), N-[2-Fluoro-4-({2-[4-(4-methylpiperazin-1-yl)piperidin-1-yl]carbonylaminopyridin-4-yl}oxy)phenyl]-N'-(4-fluorophenyl)cyclopropane-1,1-dicarboxamide(2R,3R)-tartrate, MK8033 (Merck, Inc.), BMS777607 (Bristol-Myers Squibb, Inc.), GSK1363089/GSK089 (GlaxoSmithKline, Inc.), N-((2R)-1,4-Dioxan-2-ylmethyl)-N-methyl-N'-[3-(1-methyl-1H-pyrazol-4-yl)-5-oxo-5H-benzo[4,5]cyclohepta [1,2-b]pyridin-7-yl]sulfamide, PF-04254644 (Pfizer, Inc.), N-(1,3-Benzodioxol-5-ylmethyl)-4-benzofuro[3,2-D]pyrimidin-4-yl-1-piperazinecarbothio amide, carboplatin, 9S-(9α,10β,12α))-2,3,9,10,11,12-hexahydro-10-hydroxy-10-(methoxycarbonyl)-9-methyl-9,12-epoxy-1H-diindolo[1,2,3-fg:3',2',1'-kl]pyrrolo[3,4-i]-[1,6]benzodiazocin-1-one, oxaliplatin, etoposide (VP-16, Etopophos, VePesid), ARQ197 (ArQule, Inc.), teniposide (VM-26, Vumon) topotecan (Hycamtin), irinotecan (Camptosar), carmustine (BCNU), TAK-701 (Millenium/Taked, Inc.), Amix, ab1.29.1, ab1.74.1, ab1.75.1, ab2.4.4, ab2.12.1, HGF splice variants NK2 and NK4, uncleaveable HGF, CGEN241 (Compugen, Inc.), OA-5D5 (Genentech, Inc.), isolated c-met Sema domain, DN30 (Metheresis Translational Res., Inc.), L2G7 (Galaxy Biotech, Inc.), PF-02341066 (Pfizer, Inc.), lomustine (CCNU), JNJ-38877605 (Johnson & Johnson, Inc.), streptozocin (Zanosar), 1,3-Dihydro-3-[(3,5-dimethyl-1H-pyrrol-2-yl)methylene]-2H-indol-2-one, SGX-523 (SGX Pharmaceuticals, Inc.), aminoglutethimide, formestane, (3Z)-N-(3-Chlorophenyl)-3-({3,5-dimethyl-4-[(4-methylpiperazin-1-yl)carbonyl]-1H-pyrrol-2-yl}methylene)-N-methyl-2-oxo-2,3-dihydro-1H-indole-5-sulfonamide, XL880 (Exelixis, Inc.), anastrozole, MGCD-265 (MethylGene, Inc.), genestein, erythropoietin, ibritumomab (Zevalin^{®}), levamisole, asparaginase (Elspar), 2-(4-(1-(quinolin-6-ylmethyl)-1H-[1,2,3]triazolo[4,5-b]pyrazin-6-yl)-1H-pyrazol-1-yl)ethanol, procarbazine (Matulane) and imatinib mesylate (Gleevec), bevamizumab, avastin, 3-[(1R)-1-(2,6-dichloro-3-fluorophenyl)ethoxy]-5-(1-piperidin-4-ylpyrazol-4-yl)pyridin-2-amine, angiostatin and celecoxib.

This invention relates also to drug combinations comprising or consisting of:
(i) at least one pharmaceutically active agent A, selected from antiproliferative agents and
(ii) at least one pharmaceutically active agent B, selected from supporting agents able to increase the antiproliferative effect of agent A and/or able to decrease drug resistance to agent A and
or
(i*) at least one pharmaceutically active agent B, selected from antiproliferative agents and
(ii*) at least one pharmaceutically active agent A, selected from supporting agents able to increase the antiproliferative effect of agent B and/or able to decrease drug resistance to agent B and

This invention relates also to pharmaceutical composition comprising or consisting of:
(i) at least one pharmaceutically active agent A, selected from antiproliferative agents and
(ii) at least one pharmaceutically active agent B, selected from supporting agents able to increase the antiproliferative effect of agent A and/or able to decrease drug resistance to agent A and
(iii) at least one pharmaceutically acceptable carrier, excipient or solvent
or
(l*) at least one pharmaceutically active agent B, selected from antiproliferative agents and
(ii*) at least one pharmaceutically active agent A, selected from supporting agents able to increase the antiproliferative effect of agent B and/or able to decrease drug resistance to agent B and
(iii*) at least one pharmaceutically acceptable carrier, excipient or solvent.

The drug combination or pharmaceutical composition can be used for the treatment of diseases involving cell proliferation, migration or apoptosis of myeloma cells, or angiogenesis, and especially for inhibiting tumor growth, survival and metastasis.

This invention relates also to the sequential or simultaneous use of effective amounts of
(i) at least one pharmaceutically active agent A, selected from antiproliferative agents and
(ii) at least one pharmaceutically active agent B, selected from supporting agents able to increase the antiproliferative effect of agent A and/or able to decrease drug resistance to agent A
or
(l*) at least one pharmaceutically active agent B, selected from antiproliferative agents and
(ii*) at least one pharmaceutically active agent A, selected from supporting agents able to increase the antiproliferative effect of agent B and/or able to decrease drug resistance to agent B
for the treatment of proliferative diseases
or
for the manufacture of a pharmaceutical composition for the treatment of proliferative diseases.

The proliferative diseases involving cell proliferation, migration or apoptosis of myeloma cells, or angiogenesis, tumor growth, tumor survival and metastasis formation.

The invention also relates to the use of
(i) at least one pharmaceutically active agent A, selected from antiproliferative agents and
(ii) at least one pharmaceutically active agent B, selected from supporting agents able to increase the antiproliferative effect of agent A and/or able to decrease drug resistance to agent A
or
(l*) at least one pharmaceutically active agent B, selected from antiproliferative agents and
(ii*) at least one pharmaceutically active agent A, selected from supporting agents able to increase the antiproliferative effect of agent B and/or able to decrease drug resistance to agent B
for the treatment of diseases involving cell proliferation, migration or apoptosis of myeloma cells, or angiogenesis, and especially for treating cancer, inhibiting tumor growth and/or preventing formation of metastases and more especially for overcoming or decreasing drug resistance.
or
for the manufacture of a pharmaceutical composition for the treatment of diseases involving cell proliferation, migration or apoptosis of myeloma cells, or angiogenesis, and especially for treating cancer, inhibiting tumor growth and/or preventing formation of metastases and more especially for overcoming or decreasing drug resistance.

Thus, the beneficial efficacy of the method or use in accordance with the invention are mainly based on the additive and synergistic effects of the combined treatment, or to an improved tolerability of the treatment by the patient due, for example, to the administration of lower doses of the therapeutic agents involved.

"Co-administration" or "combined treatment" or "combination treatment" to a person in need of such treatment with the selected active agent A and second agent B is meant to include administration and/or treatment sequential in time or simultaneous administration and/or treatment. For sequential administration and/or treatment, the selected agent A can be administered before or after administration of the selected agent B.

According to the present invention, a synergistic combined pharmaceutical composition is meant to comprise an amount of the selected agent A, or of a polymorph, metabolite or pharmaceutically acceptable salt of this active agent A, and an amount of the further selected agent B or of a polymorph, metabolite or pharmaceutically acceptable salt of this active agent B, wherein the individual administration of the pharmaceutical agents alone is insufficient to achieve the therapeutic effect or any therapeutic effect at all, compared to the therapeutic effect achieved by the administration of the combination of said pharmaceutical agents, and wherein the combined effects of the amounts of the pharmaceutical agents is greater than the sum of the therapeutic effects achievable with the amounts of the individual pharmaceutical agents. The selected agent A is most preferably selected from 2-cyano-3-(3,4-dihydroxyphenyl)-N-(phenylmethyl)-2-propenamide, N-[4-[(3-Chloro-4-fluorophenyl)amino]-7-[[(3"S")-tetrahydro-3-furanyl]oxy]-6-quinazolinyl]-4(dimethyl amino)-2-butenamide, [4-[[1-(3-fluorophenyl)methyl]-1H-indazol-5-ylamino]-5-methylpyrrolo[2,1-f][1,2,4]triazin-6-yl]carbamic acid (3S)-3-morpholinylmethylester, 7-(4-(3-ethynylphenylamino)-7-methoxyquinazolin-6-yloxy)-N-hydroxyheptanamide, 4-[(3-Bromophenyl)amino]-6,7-dimethoxyquinazoline hydrochloride, (E)-N-(4-((3-chloro-4-fluorophenyl)amino)-3-cyano-7-ethoxyquinolin-6-yl)-4-(dimethylamino)but-2-enamide, Vandetanib, N-(3-(5-chloro-2-(4-(4-methylpiperazin-1-yl)phenylamino)pyrimidin-4-yloxy)phenyl)acrylamide, N-(3-(5-chloro-2-(2-methoxy-4-(4-methylpiperazin-1-yl)phenylamino) pyrimidin-4-yloxy)phenyl)acrylamide, N-(3-(5-chloro-2-(4-(4-methylpiperazin-1-yl)phenylamino)pyrimidin-4-ylthio)phenyl)acrylamide, N-[3-chloro-4-[(3-fluorophenyl)methoxy]phenyl]-6-[5-[(2-methylsulfonylethyl-amino)methyl]-2-furyl]quinazolin-4-amine, 4-(R)-phenethylamino-6-(hydroxyl)phenyl-7H-pyrrolo[2.3-d]-pyrimidine, (R)-6-(4-((4-methylpiperazin-1-yl)methyl)phenyl)-N-(1-phenylethyl)-7H-pyrrolo[2,3-d]pyrimidin-4-amine, N-[-4-[(3-Chloro-4-fluorophenyl)-amino]-7-[3-(4-morpholinyl)propoxy]-6-quinazolinyl]-2-propenamide and N-(3-chlorophenyl)-6,7-dimethoxy-4-quinazolinamine.

The present invention also relates to a kit for the treatment of diseases involving cell proliferation, migration or apoptosis of myeloma cells, or angiogenesis, comprising a therapeutically effective amount of a selected agent A or of a polymorph, metabolite or pharmaceutically acceptable salt thereof, and a selected agent B or of a polymorph, metabolite or pharmaceutically acceptable salt thereof, characterized in that the selected agent A is comprised within a first compartment and the further selected agent B is comprised within a second compartment, such that the administration to a patient in need thereof can be simultaneous. Most preferred agents B used within the present invention are (2R)-1-[[5-[(Z)-[5-[[(2,6-Dichlorophenyl)methyl]sulfonyl]-1,2-dihydro-2-oxo-3H-indol-3ylidene]methyl]-2,4-dimethyl-1H-pyrrol-3-yl]carbonyl]-2-(1-pyrrolidinylmethyl)pyrrolidine, Amg-208 (Amgen, Inc.), (3Z)-N-(3-Chlorophenyl)-3-({3,5-dimethyl-4-[(4-methylpiperazin-1-yl)carbonyl]-1H-pyrrol-2-yl}methylene)-N-methyl-2-oxo-2,3-dihydro-1H-indole-5-sulfonamide, Genestein, (9S-(9α,10β,12α))-2,3,9,10,11,12-hexahydro-10-hydroxy-10-(methoxycarbonyl)-9-methyl-9,12-epoxy-1H-diindolo[1,2,3-fg:3',2',1'-kl]pyrrolo[3,4-i][1,6]benzodiazocin-1-one, (1,3-Dihydro-3-[(3,5-dimethyl-1H-pyrrol-2-yl)methylene]-2H-indol-2-one, Geldanamycin, JNJ-38877605 (Johnson & Johnson, Inc.), MGCD-265 (MethylGene, Inc.), 2-(4-(1-(quinolin-6-ylmethyl)-1H-[1,2,3]triazolo[4,5-b]pyrazin-6-yl)-1H-pyrazol-1-yl)ethanol, 3(-[(1R)-1-(2,6-dichloro-3-fluorophenyl)ethoxy]-5-(1-piperidin-4-ylpyrazol-4-yl)pyridin-2-amine, TAK-701 (Millenium/Taked, Inc.), Amix, ab1.29.1, ab1.74.1, ab1.75.1, ab2.4.4, ab2.12.1, HGF splice variants NK2 and NK4, uncleaveable HGF, CGEN241 (Compugen, Inc.), OA-5D5 (Genentech, Inc.), isolated c-met Sema domain, DN30 (Metheresis Translational Res., Inc.), L2G7 (Galaxy Biotech, Inc.), PF-02341066 (Pfizer, Inc.), (2R)-1-[[5-[(Z)-[5-[[(2,6-Dichlorophenyl)methyl]sulfonyl]-1,2-dihydro-2-oxo-3H-indol-3-ylidene]methyl]-2,4-dimethyl-1H-pyrrol-3-yl]carbonyl]-2-(1-pyrrolidinylmethyl) pyrrolidine, SGX-523 (SGX Pharmaceuticals, Inc.), (3Z)-N-(3-Chlorophenyl)-3-({3,5-dimethyl-4-[(4-methyl piperazin-1-yl)carbonyl]-1H-pyrrol-2-yl}methylene)-N-methyl-2-oxo-2,3-dihydro-1H-indole-5-sulfonamide, XL880 (Exelixis, Inc.), AMG102 (Amgen, Inc.), SCH900105 (AV-299) (Aveo Pharmaceuticals, Inc.), ARQ197 (ArQule, Inc.), XL184 (Exelixis, Inc.), N-((2R)-1,4-Dioxan-2-ylmethyl)-N-methyl-N'-[3-(1-methyl-1H-pyrazol-4-yl)-5-oxo-5H-benzo[4,5]cyclohepta[1,2-b]pyridin-7-yl]sulfamide, RP1040 (Incozen, Inc.), CEP-A (Cephalon, Inc.), N-[2-Fluoro-4-({2-[4-(4-methylpiperazin-1-yl)piperidin-1-yl]carbonylaminopyridin-4-yl}oxy)phenyl]-N'-(4-fluorophenyl)cyclopropane-1,1-dicarboxamide(2R,3R)-tartrate, MK8033 (Merck, Inc.), BMS777607 (Bristol-Myers Squibb, Inc.), GSK1363089/GSK089 (GlaxoSmithKline, Inc.), PF-04254644 (Pfizer, Inc.), N-(1,3-Benzodioxol-5-ylmethyl)-4-benzofuro[3,2-D]pyrimidin-4-yl-1-piperazinecarbothio amide.

The combination treatment in accordance with the present invention is especially efficient for inhibiting tumor growth, survival and metastasis as well as for treating drug resistant cancers and tumors.

The drug combination according to the invention is especially useful in cancer therapy. It is not limited to particular carcinomas, tumor or cancer types. The indication is especially based on the type of the used antiangiogenic and/ or cytotoxic and/or cytostatic compound, i.e. the cancer types where these antiangiogenic and/ or cytotoxic and/or cytostatic agent has been used so far as a single substance.

According to the invention, the drug combination as well as the combined treatment is especially useful in case of the following tumors or respectively cancer types: acute lymphoblastic leukemia, acute myeloid leukemia, adrenocortical carcinoma, aids-related cancers, aids-related lymphoma, anal cancer, appendix cancer, astrocytomas, atypical teratoid/rhabdoid tumor, basal cell carcinoma, bile duct cancer, bladder cancer, bone cancer, osteosarcoma and malignant fibrous histiocytoma, brain stem glioma, brain tumor, central nervous system atypical teratoid/rhabdoid tumor, astrocytomas, craniopharyngioma, ependymoblastoma, ependymoma, medulloblastoma, medulloepithelioma, pineal parenchymal tumors, supratentorial primitive neuroectodermal tumors and pineoblastoma, brain and spinal cord tumors, breast cancer, bronchial tumors, burkitt lymphoma, carcinoid tumor, carcinoid tumor, gastrointestinal tumor, central nervous system atypical teratoid/rhabdoid tumor, central nervous system (cns) lymphoma, cervical cancer, chordoma, chronic lymphocytic leukemia, chronic myelogenous leukemia, chronic myeloproliferative disorders, colon cancer, colorectal cancer, craniopharyngioma, cutaneous t-cell lymphoma, mycosis fungoides and sézary syndrome, endometrial cancer, ependymoblastoma, ependymoma, esophageal cancer, esthesioneuroblastoma, ewing sarcoma family of tumors, extracranial germ cell tumor, extragonadal germ cell tumor, extrahepatic bile duct cancer, intraocular melanoma, retinoblastoma, gallbladder cancer, gastric (stomach) cancer, gastrointestinal carcinoid tumor, gastrointestinal stromal tumor (gist), gastrointestinal stromal cell tumor, germ cell tumor (extracranial), germ cell tumor (extragonadal), germ cell tumor (ovarian), gestational trophoblastic tumor, glioma, hairy cell leukemia, head and neck cancer, heart cancer, hepatocellular (liver) cancer, histiocytosis, hodgkin lymphoma, hypopharyngeal cancer, intraocular melanoma, islet cell tumors (endocrine pancreas), kaposi sarcoma, kidney (renal cell) cancer, kidney cancer, langerhans cell histiocytosis, laryngeal cancer, leukemia (acute lymphoblastic), leukemia (acute myeloid), leukemia (chronic lymphocytic), leukemia (chronic myelogenous), leukemia (hairy cell), lip and oral cavity cancer, liver cancer, lung cancer (non-small cell), lung cancer (small cell), lymphoma (aids-related), lymphoma (burkitt), lymphoma (cutaneous t-cell), mycosis fungoides and sézary syndrome, lymphoma (hodgkin), lymphoma (non-hodgkin), lymphoma primary central nervous system (cns), macroglobulinemia, malignant fibrous histiocytoma of bone and osteosarcoma, medulloblastoma, medulloepithelioma, melanoma, melanoma intraocular (eye), merkel cell carcinoma, mesothelioma, metastatic squamous neck cancer with occult primary, mouth cancer, multiple endocrine neoplasia syndromes, multiple myeloma/plasma cell neoplasm, mycosis fungoides, myelodysplastic syndromes, myelodysplastic/myeloproliferative neoplasms, myelogenous leukemia, myeloid leukemia, myeloma (multiple), myeloproliferative disorders, nasal cavity and paranasal sinus cancer, nasopharyngeal cancer, neuroblastoma, non-hodgkin lymphoma, non-small cell lung cancer, oral cancer, oral cavity cancer, oropharyngeal cancer, osteosarcoma and malignant fibrous histiocytoma of bone, ovarian cancer, ovarian epithelial cancer, ovarian germ cell tumor, ovarian low malignant potential tumor, pancreatic cancer, papillomatosis, paranasal sinus and nasal cavity cancer, parathyroid cancer, penile cancer, pharyngeal cancer, pineal parenchymal tumors of intermediate differentiation, pineoblastoma and supratentorial primitive neuroectodermal tumors, pituitary tumor, plasma cell neoplasm/multiple myeloma, pleuropulmonary blastoma, pregnancy and breast cancer, primary central nervous system (cns) lymphoma, prostate cancer, rectal cancer, renal cell (kidney) cancer, transitional cell cancer, respiratory tract cancer, retinoblastoma, rhabdomyosarcoma, salivary gland cancer, sarcoma (ewing sarcoma), sarcoma (kaposi), sarcoma (soft tissue), sarcoma (uterine), sézary syndrome, skin cancer (nonmelanoma), skin cancer (melanoma), skin carcinoma (merkel cell), small cell lung cancer, small intestine cancer, soft tissue sarcoma, squamous cell carcinoma, squamous neck cancer, stomach (gastric) cancer, supratentorial primitive neuroectodermal tumors, t-cell lymphoma, mycosis fungoides and sézary syndrome, testicular cancer, throat cancer, thymoma and thymic carcinoma, thyroid cancer, transitional cell cancer of the renal pelvis and ureter, trophoblastic tumor, gestational, ureter and renal pelvis, transitional cell cancer, urethral cancer, uterine cancer, endometrial, uterine sarcoma, vaginal cancer, vulvar cancer, waldenström macroglobulinemia and wilms tumor.

The combined use of the agent A selected from chlorethamine, treosulfan, N-(3-chlorophenyl)-6,7-dimethoxy-4-quinazolinamine and tamoxifen, and the use of agent B selected from oxaliplatin, XL880 (Exelixis, Inc.), endostatin and celecoxib is especially preferred for the treatment of Hodgkin's disease, small cell and non small cell bronchial carcinoma and mycosis fungoides.

The combined use of the agent A selected from cyclophosphamide, chlorambucil (Leukeran), fludarabine (Fludara), 7-(4-(3-ethynylphenylamino)-7-methoxyquinazolin-6-yloxy)-N-hydroxyheptanamide and N-[4-[(3-Chloro-4-fluorophenyl)amino]-7-[[(3"S")-tetrahydro-3-furanyl]oxy]-6-quinazolinyl]-4(dimethylamino)-2-butenamide, and the use of agent B selected from streptozocin (Zanosar), (3Z)-N-(3-Chlorophenyl)-3-({3,5-dimethyl-4-[(4-methylpiperazin-1-yl)carbonyl]-1H-pyrrol-2-yl}methylene)-N-methyl-2-oxo-2,3-dihydro-1H-indole-5-sulfonamide, procarbazine (Matulane), combrestatin and AMG102 (Amgen, Inc.) is especially preferred for the treatment of breast carcinoma, soft tissue tumors, Hodgkin's disease, Non-Hodgkin's lymphomas, Ewing's tumors, medulloblastomas, acute and chronic myeloid leukemia, acute and chronic lymphatic leukemia, retinoblastoma and neuroblastomas.

The combined use of the agent A selected from trofosfamide, N-(3-(5-chloro-2-(4-(4-methylpiperazin-1-yl)phenylamino)pyrimidin-4-ylthio)phenyl)acrylamide, panitumumab, chlorethamine, trofosfamide, 4-phenethylamino-6-(yderoxyl)phenyl-7H-pyrrolo(2,3-d)pyrimidine, cytosine arabinoside and deoxycoformycin (Pentostatin, Nipent), and the use of agent B selected from (R,Z)-5-((2,6-dichlorobenzyl)sulfonyl)-3-((3,5-dimethyl-4-(2-(pyrrolidin-1-ylmethyl)pyrrolidine-1-carbonyl)-1H-pyrrol-2-yl)methylene)indolin-2-one, vindesine, etoposide, 2-(4-(1-(quinolin-6-ylmethyl)-1H-[1,2,3]triazolo[4,5-b]pyrazin-6-yl)-1H-pyrazol-1-yl)ethanol, angiostatin and vitaxin is especially preferred for the treatment of ovarian carcinoma, breast carcinoma, small cell bronchial carcinoma and testicle cancer.

The combined use of the agent A selected from ifosfamide, mechlorethamine hydrochloride (Mustargen), busulfan (Myleran), thiotepa, cetuximab, amsacrine and 4-[(3-Bromophenyl)amino]-6,7-dimethoxyquinazoline hydrochloride, and the use of agent B selected from AMG-208 (Amgen, Inc.), mitomycin C (Mitomycin), plicamycin (Mithracin), amsacrine, actinomycin D and 9S-(9α,10β,12α))-2,3,9,10,11,12-hexahydro-10-hydroxy-10-(methoxycarbonyl)-9-methyl-9,12-epoxy-1H-diindolo[1,2,3-fg:3',2',1'-kl]pyrrolo[3,4-i][1,6]benzodiazocin-1-one, is especially preferred for the treatment of soft tissue tumors, osteosarkom (bone cancer), Non-Hodgkin's lymphomas, Ewing's tumors and germ cell tumor.

The combined use of the agent A selected from melphalan, capecitabine (Xeloda), lapatinib, chlorotrianisene (TACE), diethylstilbestrol (DES)] and aromatase inhibitors [anastrozole (Arimidex)], and the use of agent B selected from SCH900105 (AV-299) (Aveo Pharmaceuticals, Inc.), XL184 (Exelixis, Inc.), N-((2R)-1,4-Dioxan-2-ylmethyl)-N-methyl-N'-[3-(1-methyl-1H-pyrazol-4-yl)-5-oxo-5H-benzo[4,5]cyclohepta[1,2-b]pyridin-7-yl]sulfamide and PF-04254644 (Pfizer, Inc.) is especially preferred for the treatment of multiple myeloma, ovarian cancer and malignant melanoma.

The combined use of the agent A selected from chlorambucil, glucocorticoids (prednisone), estramustine (Emcyt), N-(3-(5-chloro-2-(2-methoxy-4-(4-methylpiperazin-1-yl)phenylamino)pyrimidin-4-yloxy)phenyl)acrylamide and nimustine, and the use of agent B selected from camptothecin, MGCD-265 (MethylGene, Inc.) and N-(1,3-Benzodioxol-5-ylmethyl)-4-benzofuro[3,2-D]pyrimidin-4-yl-1-piperazinecarbo- thioamide is especially preferred for the treatment of Leukemias, malignant lymphomas and Morbus Waldenström.

The combined use of the agent A selected from busulfan, dacarbazine and [4-[[1-(3-fluorophenyl)methyl]-1H-indazol-5-ylamino]-5-methylpyrrolo[2,1-f][1,2,4]triazin-6-yl]carbamic Acid, and the use of agent B selected from mitoxantrone (Novantrone), AMG102 (Amgen, Inc.) and dactinomycin is especially preferred for the treatment of chronic myeloid leukemia (CML).

The combined use of the agent A selected from thiotepa, goserelin acetate (Zoladex), floxuridine (FUDR) and cladribine, and the use of agent B selected from etoposide (VP-16, Etopophos, VePesid), exemestane and 3-[(1R)-1-(2,6-dichloro-3-fluorophenyl)ethoxy]-5-(1-piperidin-4-ylpyrazol-4-yl)pyridin-2-amine, is especially preferred for the treatment of breast carcinoma, bladder carcinoma and ovarian carcinoma.

The combined use of the agent A selected from carmustine, progestine (Medoxyprogesterone acetate [(Depo-provera)] and 2-cyano-3-(3,4-dihydroxyphenyl)-N-(phenylmethyl)-2-propenamide, and the use of agent B selected from irinotecan (Camptosar), formestane, Neovastat and JNJ-38877605 (Johnson & Johnson, Inc.), is especially preferred for the treatment of brain cancer including glioma, glioblastoma multiforme, medulloblastoma and astrocytoma; multiple myeloma and lymphoma (Hodgkin's and non-Hodgkin).

The combined use of the agent A selected from 2-cyano-3-(3,4-dihydroxyphenyl)-N-(phenylmethyl)-2-propenamide, N-[4-[(3-Chloro-4-fluorophenyl)amino]-7-[[(3"S")-tetrahydro-3-furanyl]oxy]-6-quinazolinyl]-4(dimethyl amino)-2-butenamide, [4-[[1-(3-fluorophenyl)methyl]-1H-indazol-5-ylamino]-5-methylpyrrolo[2,1-f][1,2,4]triazin-6-yl]carbamic acid (3S)-3-morpholinyl-methylester,7-(4-(3-ethynylphenylamino)-7-methoxyquinazolin-6-yloxy)-N-hydroxyheptanamide, 4-[(3-Bromophenyl)amino]-6,7-dimethoxyquinazoline hydrochloride, (E)-N-(4-((3-chloro-4-fluorophenyl)amino)-3-cyano-7-ethoxyquinolin-6-yl)-4-(dimethylamino) but-2-enamide, Vandetanib, (N-(3-(5-chloro-2-(4-(4-methylpiperazin-1-yl)phenylamino)pyrimidin-4-yloxy)phenyl)acryl amide, N-(3-(5-chloro-2-(2-methoxy-4-(4-methylpiperazin-1-yl)phenylamino) pyrimidin-4-yloxy)phenyl)acrylamide, N-(3-(5-chloro-2-(4-(4-methylpiperazin -1-yl)phenylamino) pyrimidin-4-ylthio)phenyl)acrylamide, Lapatinib, 4-phenethylamino-6-(yderoxyl)phenyl-7H-pyrrolo(2,3-d)pyrimidine, (R)-6-(4-((4-methylpiperazin-1-yl)methyl)phenyl)-N-(1-phenylethyl)-7H-pyrrolo[2,3-d]pyrimidin-4-amine, N-[-4-[(3-Chloro-4-fluorophenyl)amino]-7-[3-(4-morpholinyl) propoxy]-6-quinazolinyl]-2-propenamide and N-(3-chlorophenyl)-6,7-dimethoxy-4-quinazolinamine and the use of agent B selected from (R,Z)-5-((2,6-dichlorobenzyl)sulfonyl)-3-((3,5-dimethyl-4-(2-(pyrrolidin-1-ylmethyl)pyrrolidine-1-carbonyl)-1H-pyrrol-2-yl)methylene)indolin-2-one, (3Z)-N-(3-Chlorophenyl)-3-({3,5-dimethyl-4-[(4-methylpiperazin-1-yl)carbonyl]-1H-pyrrol-2-yl}methylene)-N-methyl-2-oxo-2,3-dihydro-1H-indole-5-sulfonamide), (9S-(9a,10β,12α))-2,3,9,10,11,12-hexahydro-10-hydroxy-10-(methoxycarbonyl)-9-methyl-9,12-epoxy-1H-diindolo[1,2,3-fg:3',2',1'-kl]pyrrolo[3,4-i][1,6]benzodiazocin-1-one,1,3-Dihydro-3-[(3,5-dimethyl-1H-pyrrol-2-yl)methylene]-2H-indol-2-one, TAK-701 (Millenium/Taked, Inc.), Amix, ab1.29.1, ab1.74.1, ab1.75.1, ab2.4.4, ab2.12.1, HGF splice variants NK2 and NK4, uncleaveable HGF, CGEN241 (Compugen, Inc.), OA-5D5 (Genentech, Inc.), isolated c-met Sema domain, DN30 (Metheresis Translational Res., Inc.), L2G7 (Galaxy Biotech, Inc.), PF-02341066 (Pfizer, Inc.), Genestein, Geldanamycin, Amg-208 (Amgen, Inc.), JNJ-38877605 (Johnson & Johnson, Inc.), MGCD-265 (MethylGene, Inc.), 2-(4-(1-(quinolin-6-ylmethyl)-1H-[1,2,3]triazolo[4,5-b]pyrazin-6-yl)-1H-pyrazol-1-yl)ethanol, 3(-[(1R)-1-(2,6-dichloro-3-fluorophenyl) ethoxy]-5-(1-piperidin-4-ylpyrazol-4-yl)pyridin-2-amine), SGX-523 (SGX Pharmaceuticals, Inc.), (3Z)-N-(3-Chlorophenyl)-3-({3,5-dimethyl-4-[(4-methylpiperazin-1-yl)carbonyl]-1H-pyrrol-2-yl}methylene)-N-methyl-2-oxo-2,3-dihydro-1H-indole-5-sulfonamide, RP1040 (Incozen, Inc.), CEP-A (Cephalon, Inc.), N-[2-Fluoro-4-({2-[4-(4-methylpiperazin-1-yl)piperidin-1-yl]carbonylaminopyridin-4-yl}oxy)phenyl]-N'-(4-fluorophenyl)cyclopropane-1,1-dicarboxamide(2R,3R)-tartrate, MK8033 (Merck, Inc.), BMS777607 (Bristol-Myers Squibb, Inc.), GSK1363089/GSK089 (GlaxoSmithKline, Inc.), XL880 (Exelixis, Inc.), AMG102 (Amgen, Inc.), SCH900105 (AV-299) (Aveo Pharmaceuticals, Inc.), ARQ197 (ArQule, Inc.), XL184 (Exelixis, Inc.), N-((2R)-1,4-Dioxan-2-ylmethyl)-N-methyl-N'-[3-(1-methyl-1H-pyrazol-4-yl)-5-oxo-5H-benzo[4,5]cyclohepta[1,2-b]pyridin-7-yl]sulfamide, PF-04254644 (Pfizer, Inc.), N-(1,3-Benzodioxol-5-ylmethyl)-4-benzofuro[3,2-D]pyrimidin-4-yl-1-piperazinecarbothioamide is especially preferred for the treatment of breast cancer, kidney cancer, liver cancer and head and neck cancer.

The combined use of the agent A selected from pentostatin, (E)-N-(4-((3-chloro-4-fluorophenyl)amino)-3-cyano-7-ethoxyquinolin-6-yl)-4-(dimethyl amino)but-2-enamide and megestrol acetate (Megacel), and the use of agent B selected from lomustine, bleomycin (Blenoxane), teniposide (VM-26, Vumon) topotecan (Hycamtin) and 1,3-Dihydro-3-[(3,5-dimethyl-1H-pyrrol-2-yl)methylene]-2H-indol-2-one is especially preferred for the treatment of brain tumors.

The combined use of the agent A selected from dacarbazine, 6-mercaptopurine, N-[-4-[(3-Chloro-4-fluorophenyl)amino]-7-[3-(4-morpholinyl)propoxy]-6-quinazolinyl]-2-propenamide and gemcitabine (Gemzar), and the use of agent B selected from letrozole, anastrozole, tirapazamine and endostatin is especially preferred for the treatment of malignant melanoma, Hodgkin lymphoma, sarcoma and islet cell carcinoma of the pancreas.

The combined use of the agent A selected from procarbazine, N-(3-(5-chloro-2-(4-(4-methylpiperazin-1-yl)phenylamino)pyrimidin-4-yloxy)phenyl)acrylamide, and the use of agent B selected from carmustine (BCNU), dactinomycin and genestein is especially preferred for the treatment of Hodgkin's lymphoma and brain cancers (such as Glioblastoma multiforme).

The combined use of the agent A selected from temozolomide, pentostatin, bexarotene and tretinoin, and the use of agent B selected from levamisole and asparaginase (Elspar), is especially preferred for the treatment of astrocytoma, glioblastoma and brain tumor.

The combined use of the agent A selected from treosulfan, (R)-6-(4-((4-methylpiperazin-1-yl)methyl)phenyl)-N-(1-phenylethyl)-7H-pyrrolo[2,3-d]pyrimidin-4-amine and, LHRH (buserelin, goserelin, leuprorelin, triptorelin), and the use of agent B selected from topotecan, aminoglutethimide and epirubicin (4-epi-adriamycin) is especially preferred for the treatment of ovarian cancer.

The combined use of the agent A selected from estramustine, procarbazine, cyproterone acetate and toremifen and the use of agent B selected from doxorubicin (adriamycin) and avastin is especially preferred for the treatment of prostate cancer.

The pharmaceutical composition can also be administered in form of their pharmaceutically active salts optionally using substantially nontoxic pharmaceutically acceptable carrier, excipients, adjuvants or diluents. The medications of the present invention are prepared in a conventional solid or liquid carrier or diluents and a conventional pharmaceutically-made adjuvant at suitable dosage level in a known way. The preferred preparations and compositions are in administratable form which is suitable for oral application. These administratable forms, for example, include pills, tablets, film tablets, coated tablets, capsules, powders and deposits. Other than oral administratable forms are also possible. The inventive compounds or pharmaceutical preparations or compositions containing said compounds may be administered by any appropriate means, including but not limited to inhalation, injection (intravenous, intraperitoneal, intramuscular, subcutaneous) by absorption through epithelial or mucocutaneous linings (oral mucosa, rectal and vaginal epithelial linings, nasopharyngial mucosa, intestinal mucosa); orally, rectally, transdermally, topically, intradermally, intragastrically, intracutaneously, intravaginally, intravasally, intranasally, intrabuccally, percutaneously, sublingually, or any other means available within the pharmaceutical arts.

Within the disclosed methods the pharmaceutical compositions of the present invention, containing at least one pharmaceutically active agent selected from antiproliferative agents and at least one pharmaceutically active agent selected from supporting agents or pharmaceutically acceptable salts thereof as an active ingredient will typically be administered in admixture with suitable carrier materials suitably selected with respect to the intended form of administration, i.e. oral tablets, capsules (either solid-filled, semi-solid filled or liquid filled), powders for constitution, oral gels, elixirs, dispersible granules, syrups, suspensions, and the like, and consistent with conventional pharmaceutical practices. For example, for oral administration in the form of tablets or capsules, the active ingredient may be combined with any oral nontoxic pharmaceutically acceptable inert carrier, such as lactose, starch, sucrose, cellulose, magnesium stearate, dicalcium phosphate, calcium sulfate, talc, mannitol, ethyl alcohol (liquid forms) and the like. Moreover, when desired or needed, suitable binders, lubricants, disintegrating agents and coloring agents may also be incorporated in the mixture. Powders and tablets may be comprised of from about 5 to about 95 percent inventive composition.

Suitable binders include starch, gelatin, natural sugars, corn sweeteners, natural and synthetic gums such as acacia, sodium alginate, carboxymethyl-cellulose, polyethylene glycol and waxes. Among the lubricants that may be mentioned for use in these dosage forms, boric acid, sodium benzoate, sodium acetate, sodium chloride, and the like. Disintegrants include starch, methylcellulose, guar gum and the like. Sweetening and flavoring agents and preservatives may also be included where appropriate. Some of the terms noted above, namely disintegrants, diluents, lubricants, binders and the like, are discussed in more detail below.

Additionally, the compositions of the present invention may be formulated in sustained release form to provide the rate controlled release of any one or more of the components or active ingredients to optimize the therapeutic effects, i.e. antihistaminic activity and the like. Suitable dosage forms for sustained release include layered tablets containing layers of varying disintegration rates or controlled release polymeric matrices impregnated with the active components and shaped in tablet form or capsules containing such impregnated or encapsulated porous polymeric matrices.

Liquid form preparations include solutions, suspensions and emulsions. As an example may be mentioned water or water-propylene glycol solutions for parenteral injections or addition of sweeteners and opacifiers for oral solutions, suspensions and emulsions. Liquid form preparations may also include solutions for intranasal administration.

Aerosol preparations suitable for inhalation may include solutions and solids in powder form, which may be in combination with a pharmaceutically acceptable carrier such as inert compressed gas, e.g. nitrogen.

For preparing suppositories, a low melting wax such as a mixture of fatty acid glycerides such as cocoa butter is first melted, and the active ingredient is dispersed homogeneously therein by stirring or similar mixing. The molten homogeneous mixture is then poured into convenient sized molds, allowed to cool and thereby solidifies.

Also included are solid form preparations which are intended to be converted, shortly before use, to liquid form preparations for either oral or parenteral administration. Such liquid forms include solutions, suspensions and emulsions.

The inventive pharmaceutical composition of the present invention may also be deliverable transdermally. The transdermal compositions may take the form of creams, lotions, aerosols and/or emulsions and can be included in a transdermal patch of the matrix or reservoir type as are conventional in the art for this purpose.

The term capsule refers to a special container or enclosure made of methyl cellulose, polyvinyl alcohols, or denatured gelatins or starch for holding or containing compositions comprising the active ingredients. Hard shell capsules are typically made of blends of relatively high gel strength bone and pork skin gelatins. The capsule itself may contain small amounts of dyes, opaquing agents, plasticizers and preservatives.

Tablet means compressed or molded solid dosage form containing the active ingredients with suitable diluents. The tablet can be prepared by compression of mixtures or granulations obtained by wet granulation, dry granulation or by compaction well known to a person skilled in the art.

Oral gels refers to the active ingredients dispersed or solubilized in a hydrophilic semi-solid matrix.

Powders for constitution refers to powder blends containing the active ingredients and suitable diluents which can be suspended in water or juices.

Suitable diluents are substances that usually make up the major portion of the composition or dosage form. Suitable diluents include sugars such as lactose, sucrose, mannitol and sorbitol, starches derived from wheat, corn rice and potato, and celluloses such as microcrystalline cellulose. The amount of diluents in the composition can range from about 5 to about 95% by weight of the total composition, preferably from about 25 to about 75%, more preferably from about 30 to about 60% by weight, and most preferably from about 40 to 50% by weight.

The term disintegrants refers to materials added to the composition to help it break apart (disintegrate) and release the medicaments. Suitable disintegrants include starches, "cold water soluble" modified starches such as sodium carboxymethyl starch, natural and synthetic gums such as locust bean, karaya, guar, tragacanth and agar, cellulose derivatives such as methylcellulose and sodium carboxymethylcellulose, microcrystalline celluloses and cross-linked microcrystalline celluloses such as sodium croscarmellose, alginates such as alginic acid and sodium alginate, clays such as bentonites, and effervescent mixtures. The amount of disintegrant in the composition can range from about 1 to about 40% by weight of the composition, preferably 2 to about 30% by weight of the composition, more preferably from about 3 to 20% by weight of the composition, and most preferably from about 5 to about 10% by weight.

Binders characterize substances that bind or "glue" powders together and make them cohesive by forming granules, thus serving as the "adhesive" in the composition. Binders add cohesive strength already available in the diluents or bulking agent. Suitable binders include sugars such as sucrose, starches derived from wheat, corn rice and potato; natural gums such as acacia, gelatin and tragacanth; derivatives of seaweed such as alginic acid, sodium alginate and ammonium calcium alginate; cellulosic materials such as methylcellulose and sodium carboxymethylcellulose and hydroxypropyl-methylcellulose; polyvinylpyrrolidone; and inorganics such as magnesium aluminum silicate. The amount of binder in the composition can range from about 1 to 30% by weight of the composition, preferably from about 2 to about 20% by weight of the composition, more preferably from about 3 to about 10% by weight, even more preferably from about 3 to about 6% by weight.

Lubricant refers to a substance added to the dosage form to enable the tablet, granules, etc. after it has been compressed, to release from the mold or die by reducing friction or wear. Suitable lubricants include metallic stearates such as magnesium stearate, calcium stearate or potassium stearate; stearic acid; high melting point waxes; and water soluble lubricants such as sodium chloride, sodium benzoate, sodium acetate, sodium oleate, polyethylene glycols and d'I-leucine. Lubricants are usually added at the very last step before compression, since they must be present on the surfaces of the granules and in between them and the parts of the tablet press. The amount of lubricant in the composition can range from about 0.05 to about 15% by weight of the composition, preferably 0.2 to about 5% by weight of the composition, more preferably from about 0.3 to about 3%, and most preferably from about 0.3 to about 1.5% by weight of the composition.

Glidents are materials that prevent caking and improve the flow characteristics of granulations, so that flow is smooth and uniform. Suitable glidents include silicon dioxide and talc. The amount of glident in the composition can range from about 0.01 to 10% by weight of the composition, preferably 0.1 % to about 7% by weight of the total composition, more preferably from about 0.2 to 5% by weight, and most preferably from about 0.5 to about 2% by weight.

Coloring agents are excipients that provide coloration to the composition or the dosage form. Such excipients can include food grade dyes and food grade dyes adsorbed onto a suitable adsorbent such as clay or aluminum oxide. The amount of the coloring agent can vary from about 0.01 to 10% by weight of the composition, preferably from about 0.05 to 6% by weight, more preferably from about 0.1 to about 4% by weight of the composition, and most preferably from about 0.1 to about 1%.

Techniques for the formulation and administration of the pharmaceutical composition of the present invention may be found in "Remington's Pharmaceutical Sciences" Mack Publishing Co., Easton PA. A suitable composition comprising the pharmaceutical composition of the invention and/or pharmaceutically acceptable salts thereof may be a solution of the composition in a suitable liquid pharmaceutical carrier or any other formulation such as tablets, pills, film tablets, coated tablets, dragees, capsules, powders and deposits, gels, syrups, slurries, suspensions, emulsions, and the like.

A therapeutically effective dosage of the compounds of the pharmaceutical compositon refers to that amount of the compounds that result in an at least partial inhibition of proliferation. Toxicity and therapeutic efficacy of such compounds can be determined by standard pharmaceutical, pharmacological, and toxicological procedures in cell cultures or experimental animals for determining the LD50 (the dose lethal to 50% of the population) and the ED50 (the dose therapeutically effective in 50% of the population). The dose ratio between toxic and therapeutic effect is the therapeutic index and can be expressed as the ratio between LD50 and ED50. The dosage of the compound lies preferably within a range of circulating concentrations that include the ED50 with little or no toxicity. More preferably, the dosage of the compound corresponds to an effective concentration in the range of 0.1-5 µM. The actual amount of the composition administered will be dependent on the subject being treated, on the subject's weight, the severity of the affliction, the manner of administration and the judgement of the prescribing physician.

## Claims

1. Use of a combination of at least two pharmaceutically active agents A and B, wherein the pharmaceutically active agent A is selected from antiproliferative agents and agent B is selected from supporting agents able to increase the antiproliferative effect of agent A for the treatment of a proliferative disease.

2. Use according to claim 1, wherein agent A is selected from chlorethamine, cyclophosphamide, trofosfamide, ifosfamide, melphalan, mechlorethamine hydrochloride, chlorambucil, busulfan, thiotepa, 2-cyano-3-(3,4-dihydroxyphenyl)-n-(phenylmethyl)-2-propenamide, carmustine, carboplalomustine, dacarbazine, procarbazine, erlotinib, temozolomide, treosulfan, estramustine, n-(3-(5-chloro-2-(2-methoxy-4-(4-methylpiperazin-1-yl)phenylamino)pyrimidin-4-yloxy)phenyl)-acrylamide, nimustine, methotrexate, 5-fluorouracil, 6-thioguanine, 6-mercaptopurine, n-[-4-[(3-chloro-4-fluorophenyl)amino]-7-[3-(4-morpholinyl)-propoxy]-6-quinazolinyl]-2-propenamide, n-(3-chlorophenyl)-6,7-dimethoxy-4-quinazolinamine, fludarabine, floxuridine, cladribine, pentostatin, gemcitabine, cytarabine, azathioprine, raltitrexed, capecitabine, 4-phenethylamino-6-(yderoxyl)phenyl-7h-pyrrolo(2,3-d)pyrimidine, cytosine arabinoside, deoxycoformycin, lapatinib, thioguanine, 7-(4-(3-ethynylphenylamino)-7-methoxyquinazolin-6-yloxy)-n-hydroxyheptanamide, mercaptopurine, paclitaxel, docetaxel, hydroxycarbamide, imatinibe, miltefosine, n-(3-(5-chloro-2-(4-(4-methylpiperazin-1-yl)phenylamino) pyrimidin-4-yloxy)phenyl)acrylamide, amsacrine, cetuximab, 4-[(3-bromophenyl)amino]-6,7-dimethoxyquinazoline hydrochloride, pentostatin, bexarotene, tretinoin, asparaginase, trastuzumab, alemtuzumab, rituximab, n-[4-[(3-chloro-4-fluorophenyl)amino]-7-[[(3"s")-tetrahydro-3-furanyl]oxy]-6-quinazolinyl]-4(dimethylamino)-2-butenamide, glucocorticoids, hydrocortisones, gefitinib, dexamethasones, (e)-n-(4-((3-chloro-4-fluorophenyl)amino)-3-cyano-7-ethoxyquinolin-6-yl)-4-(dimethylamino)but-2-enamide, estrogens, chlorotrianisene, diethylstilbestrol, (r)-6-(4-((4-methylpiperazin-1-yl)methyl)phenyl)-n-(1-phenylethyl)-7h-pyrrolo[2,3-d]pyrimidin-4-amine, aromatase inhibitors, anastrozole, buserelin, goserelin, leuprorelin, triptorelin, n-(3-chloro-4-((3-fluorophenyl)methoxy)phenyl)-6-(5-(((2-(methylsulfonyl)ethyl)-amino)methyl)-2-furanyl)-4-quinazolinaminebis(4-methylbenzenesulfonate), flutamide, bicalutamide, leuprolide, goserelin acetate, [4-[[1-(3-fluorophenyl)methyl]-1h-indazol-5-ylamino]-5-methylpyrrolo[2,1-f] [1,2,4]triazin-6-yl]carbamic acid (3s)-3-morpholinyl methyl ester, vandetanib, cyproterone acetate, n-(3-(5-chloro-2-(4-(4-methylpiperazin-1-yl)phenylamino)pyrimidin-4-ylthio)phenyl) acrylamide, panitumumab, progestine, megestrol acetate, tamoxifen and toremifen.

3. Use according to claim 1, wherein agent B is selected from daunorubicin, doxorubicin, liposomal adriamycin, dactinomycin, mitomycin c, (r,z)-5-((2,6-dichlorobenzyl)sulfonyl)-3-((3,5-dimethyl-4-(2-(pyrrolidin-1-ylmethyl)pyrrolidine-1-carbonyl)-1h-pyrrol-2-yl)methylene)indolin-2-one, bleomycin, epirubicin idarubicin, amg102, dactinomycin, mitoxantrone, amg-208, mitomycin c, plicamycin, amsacrine, actinomycin d, vincristine, vinblastine, sch900105, vindesine, etoposide, xl184, n-((2r)-1,4-dioxan-2-ylmethyl)-n-methyl-n'-[3-(1-methyl-1h-pyrazol-4-yl)-5-oxo-5h-benzo[4,5]cyclohepta[1,2-b]pyridin-7-yl] sulfamide, pf-04254644, n-(1,3-benzodioxol-5-ylmethyl)-4-benzofuro[3,2-d]pyrimidin-4-yl-1-piperazinecarbothioamide, teniposide, cisplatin, carboplatin, rp1040, cep-a, n-[2-fluoro-4-({2-[4-(4-methylpiperazin-1-yl)piperidin-1-yl]carbonylaminopyridin-4-yl}oxy)phenyl]-n'-(4-fluorophenyl)cyclopropane-1,1-dicarboxamide(2r,3r)-tartrate, mk8033, bms777607, gsk1363089, 9s-(9a,10β,12α))-2,3,9,10,11,12-hexahydro-10-hydroxy-10-(methoxycarbonyl)-9-methyl-9,12-epoxy-1h-diindolo[1,2,3-fg:3',2',1'-kl]pyrrolo[3,4-i][1,6]benzodiazocin-1-one, oxaliplatin, vinorelbine, etoposide, arq197, teniposide, topotecan, irinotecan, carmustine, lomustine, jnj-38877605, streptozocin, 1,3-dihydro-3-[(3,5-dimethyl-1 h-pyrrol-2-yl)methylene]-2h-indol-2-one, tak-701, amix, ab1.29.1, ab1.74.1, ab1.75.1, ab2.4.4, ab2.12.1, hgf splice variants nk2 and nk4, uncleaveable hgf, cgen241, oa-5d5, isolated c-met sema domain, dn30,I2g7, pf-02341066, camptothecin, topotecan, sgx-523, irinotecan. aminoglutethimide, formestane, (3z)-n-(3-chlorophenyl)-3-({3,5-dimethyl-4-[(4-methylpiperazin-1-yl)carbonyl]-1h-pyrrol-2-yl}methylene)-n-methyl-2-oxo-2,3-dihydro-1h-indole-5-sulfonamide, x1880, exemestane, letrozole, anastrozole, mgcd-265, interleukin-2, interferon-a, genestein, erythropoietin, g-csf, ibritumomab, levamisole, asparaginase, pegaspargase, hydroxyurea, 2-(4-(1-(quinolin-6-ylmethyl)-1h-[1,2,3]triazolo[4,5-b]pyrazin-6-yl)-1h-pyrazol-1-yl)ethanol, procarbazine and imatinib mesylate, neovastat, bevamizumab, avastin, angiozyme, 3-[(1r)-1-(2,6-dichloro-3-fluorophenyl)ethoxy]-5-(1-piperidin-4-ylpyrazol-4-yl)pyridin-2-amine, endostatin, angiostatin, combrestatin, vitaxin, geldanamycin carboxyamido thiazole, celecoxib and tirapazamine.

4. Use according to claim 3, wherein agent A is selected from cyclophosphamide, mechlorethamine hydrochloride, chlorambucil, 2-cyano-3-(3,4-dihydroxyphenyl)-n-(phenylmethyl)-2-propenamide, carboplalomustine, erlotinib, temozolomide, treosulfan, n-(3-(5-chloro-2-(2-methoxy-4-(4-methylpiperazin-1-yl)phenylamino)pyrimidin-4-yloxy)phenyl)acrylamide, methotrexate, 6-thioguanine, 6-mercaptopurine, n-[-4-[(3-chloro-4-fluorophenyl)amino]-7-[3-(4-morpholinyl)propoxy]-6-quinazolinyl]-2-propenamide, n-(3-chlorophenyl)-6,7-dimethoxy-4-quinazolinamine, fludarabine, floxuridine, gemcitabine, cytarabine, capecitabine, 4-phenethylamino-6-(yderoxyl)phenyl-7h-pyrrolo(2,3-d)pyrimidine, lapatinib, thioguanine, 7-(4-(3-ethynylphenylamino)-7-methoxyquinazolin-6-yloxy)-n-hydroxyheptanamide, mercaptopurine, paclitaxel, docetaxel, hydroxycarbamide, imatinibe, miltefosine, n-(3-(5-chloro-2-(4-(4-methylpiperazin-1-yl)phenylamino)pyrimidin-4-yloxy)phenyl)acrylamide, cetuximab, amsacrine, 4-[(3-bromophenyl)amino]-6,7-dimethoxyquinazoline hydrochloride, trastuzumab, alemtuzumab, rituximab, n-[4-[(3-chloro-4-fluorophenyl)amino]-7-[[(3"s")-tetrahydro-3-furanyl]oxy]-6-quinazolinyl]-4(dimethylamino)-2-butenamide), gefitinib, (e)-n-(4-((3-chloro-4-fluorophenyl)amino)-3-cyano-7-ethoxyquinolin-6-yl)-4-(dimethylamino)but-2-enamide, (r)-6-(4-((4-methylpiperazin-1-yl)methyl) phenyl)-n-(1-phenylethyl)-7h-pyrrolo[2,3-d]pyrimidin-4-amine, estramustine, chlorotrianisene, diethylstilbestrol, lhrh, buserelin, goserelin, leuprorelin, triptorelin, n-(3-chloro-4-((3-fluorophenyl)methoxy)phenyl)-6-(5-(((2-(methylsulfonyl)ethyl)amino)methyl)-2-furanyl)-4-quinazolinaminebis(4-methylbenzenesulfonate), leuprolide, goserelin acetate, [4-[[1-(3-fluorophenyl)methyl]-1h-indazol-5-ylamino]-5-methylpyrrolo[2,1-f][1,2,4]triazin-6-yl]carbamic acid (3s)-3-morpholinylmethyl ester, n-(3-(5-chloro-2-(4-(4-methylpiperazin-1-yl)phenylamino)pyrimidin-4-ylthio)phenyl)acrylamide, panitumumab.

5. Use according to claim 3, wherein agent B is selected from (r,z)-5-((2,6-dichlorobenzyl)sulfonyl)-3-((3,5-dimethyl-4-(2-(pyrrolidin-1-ylmethyl)pyrrolidine-1-carbonyl)-1h-pyrrol-2-yl)methylene)indolin-2-one, bleomycin, epirubicin, idarubicin, amg102, dactinomycin, mitoxantrone, amg-208, plicamycin, vincristine, sch900105, vindesine, x1184, n-((2r)-1,4-dioxan-2-ylmethyl)-n-methyl-n'-[3-(1-methyl-1 h-pyrazol-4-yl)-5-oxo-5h-benzo[4,5]cyclohepta[1,2-b]pyridin-7-yl]-sulfamide, tak-701, amix, ab1.29.1, ab1.74.1, ab1.75.1, ab2.4.4, ab2.12.1, hgf splice variants nk2 and nk4, uncleaveable hgf, cgen241, oa-5d5, isolated c-met sema domain, dn30, l2g7, pf-02341066, pf-04254644, n-(1,3-benzodioxol-5-ylmethyl)-4-benzofuro[3,2-d]pyrimidin-4-yl-1-piperazinecarbothioamide, carboplatin, 9s-(90,10β,12α))-2,3,9,10,11,12-hexahydro-10-hydroxy-10-(methoxycarbonyl)-9-methyl-9,12-epoxy-1h-diindolo[1,2,3-fg:3',2',1'-kl]pyrrolo[3,4-i][1,6]benzodiazocin-1-one, oxaliplatin, etoposide, arq197, teniposide, topotecan, irinotecan, carmustine, lomustine, jnj-38877605, streptozocin, 1,3-dihydro-3-[(3,5-dimethyl-1 h-pyrrol-2-yl)methylene]-2h-indol-2-one, sgx-523, aminoglutethimide, formestane, (3z)-n-(3-chlorophenyl)-3-({3,5-dimethyl-4-[(4-methylpiperazin-1-yl)carbonyl]-1h-pyrrol-2-yl}methylene)-n-methyl-2-oxo-2,3-dihydro-1h-indole-5-sulfonamide, xl880, anastrozole, mgcd-265, genestein, erythropoietin, ibritumomab, levamisole, asparaginase, 2-(4-(1-(quinolin-6-ylmethyl)-1h-[1,2,3]triazolo[4,5-b]pyrazin-6-yl)-1h-pyrazol-1-yl)ethanol, procarbazine and imatinib mesylate, bevamizumab, rp1040, cep-a, n-[2-fluoro-4-({2-[4-(4-methylpiperazin-1-yl)piperidin-1-yl]carbonylaminopyridin-4-yl}oxy)phenyl]-n'-(4-fluorophenyl)cyclopropane-1,1-dicarboxamide(2r,3r)-tartrate, mk8033, bms777607, gsk1363089, avastin, 3-[(1r)-1-(2,6-dichloro-3-fluorophenyl)ethoxy]-5-(1-piperidin-4-ylpyrazol-4-yl)pyridin-2-amine, angiostatin and celecoxib.

6. A drug combination comprising or consisting of:
(i) at least one pharmaceutically active agent A, selected from antiproliferative agents and
(ii) at least one pharmaceutically active agent B, selected from supporting agents able to increase the antiproliferative effect of agent A and/or able to decrease drug resistance to agent A
or
(l*) at least one pharmaceutically active agent B, selected from antiproliferative agents and
(ii*) at least one pharmaceutically active agent A, selected from supporting agents able to increase the antiproliferative effect of agent B and/or able to decrease drug resistance to agent B.

7. A pharmaceutical composition comprising or consisting of:
(i) at least one pharmaceutically active agent A, selected from antiproliferative agents and
(ii) at least one pharmaceutically active agent B, selected from supporting agents able to increase the antiproliferative effect of agent A and/or able to decrease drug resistance to agent A and
(iii) at least one pharmaceutically acceptable carrier, excipient or solvent
or
(l*) at least one pharmaceutically active agent B, selected from antiproliferative agents and
(ii*) at least one pharmaceutically active agent A, selected from supporting agents able to increase the antiproliferative effect of agent B and/or able to decrease drug resistance to agent B and
(iii*) at least one pharmaceutically acceptable carrier, excipient or solvent.

8. The drug combination according to claim 6 or the pharmaceutical composition according to claim 7, wherein agent A is selected from chlorethamine, cyclophosphamide, trofosfamide, ifosfamide, melphalan, mechlorethamine hydrochloride, chlorambucil, busulfan, thiotepa, 2-cyano-3-(3,4-dihydroxyphenyl)-n-(phenylmethyl)-2-propenamide, carmustine, carboplalomustine, dacarbazine, procarbazine, erlotinib, temozolomide, treosulfan, estramustine, n-(3-(5-chloro-2-(2-methoxy-4-(4-methylpiperazin-1-yl)phenylamino)pyrimidin-4-yloxy)phenyl) acrylamide, nimustine, methotrexate, 5-fluorouracil, 6-thioguanine (thioguanine, 6-tg), 6-mercaptopurine, n-[-4-[(3-chloro-4-fluorophenyl)amino]-7-[3-(4-morpholinyl)propoxy]-6-quinazolinyl]-2-propenamide, n-(3-chlorophenyl)-6,7-dimethoxy-4-quinazolinamine, fludarabine, floxuridine, cladribine, pentostatin, gemcitabine, cytarabine, azathioprine, raltitrexed, capecitabine, 4-phenethylamino-6-(yderoxyl)phenyl-7h-pyrrolo(2,3-d)pyrimidine, cytosine arabinoside, deoxycoformycin, lapatinib, thioguanine, 7-(4-(3-ethynylphenylamino)-7-methoxyquinazolin-6-yloxy)-n-hydroxyheptanamide, mercaptopurine, paclitaxel, docetaxel, hydroxycarbamide, imatinib, miltefosine, n-(3-(5-chloro-2-(4-(4-methylpiperazin-1-yl)phenylamino)pyrimidin-4-yloxy)phenyl)acrylamide, cetuximab, amsacrine, 4-[(3-bromophenyl)amino]-6,7-dimethoxyquinazoline hydrochloride, pentostatin, bexarotene, tretinoin, asparaginase, trastuzumab, alemtuzumab, rituximab, n-[4-[(3-chloro-4-fluorophenyl)amino]-7-[[(3"s")-tetrahydro-3-furanyl]oxy]-6-quinazolinyl]-4(dimethylamino)-2-butenamide, glucocorticoids, hydrocortisones, gefitinib, dexamethasones, (e)-n-(4-((3-chloro-4-fluorophenyl)amino)-3-cyano-7-ethoxyquinolin-6-yl)-4-(dimethylamino)but-2-enamide, estrogens, estramustine, chlorotrianisene, diethylstilbestrol, (r)-6-(4-((4-methylpiperazin-1-yl)methyl)pheny))-n-(1-phenylethyl)-7h-pyrrolo[2,3-d]pyrimidin-4-amine, aromatase inhibitors, lhrh, buserelin, goserelin, leuprorelin, triptorelin, n-(3-chloro-4-((3-fluorophenyl)methoxy)phenyl)-6-(5-(((2-(methylsulfonyl)ethyl)amino)methyl)-2-furanyl)-4-quinazolinaminebis(4-methylbenzenesulfonate), flutamide, bicalutamide, leuprolide, goserelin acetate, [4-[[1-(3-fluorophenyl)methyl]-1h-indazol-5-ylamino]-5-methylpyrrolo[2,1-f][1,2,4]triazin-6-yl]carbamicacid(3s)-3-morpholinylmethylester, vandetanib, cyproterone acetate, n-(3-(5-chloro-2-(4-(4-methylpiperazin-1-yl)phenylamino)pyrimidin-4-ylthio)phenyl)acrylamide, panitumumab, progestine, megestrol acetate, tamoxifen and toremifen.

9. The drug combination according to claim 6 or the pharmaceutical composition according to claim 7, wherein agent B is selected from (r,z)-5-((2,6-dichlorobenzyl)sulfonyl)-3-((3,5-dimethyl-4-(2-(pyrrolidin-1-ylmethyl)pyrrolidine-1-carbonyl)-1h-pyrrol-2-yl)methylene)indolin-2-one, bleomycin, epirubicin, idarubicin, amg102, dactinomycin, mitoxantrone, amg-208, plicamycin, vincristine, sch900105, vindesine, xl184, n-((2r)-1,4-dioxan-2-ylmethyl)-n-methyl-n'-[3-(1-methyl-1 h-pyrazol-4-yl)-5-oxo-5h-benzo[4,5]cyclohepta[1,2-b]pyridin-7-yl]sulfamide, pf-04254644, n-(1,3-benzodioxol-5-ylmethyl)-4-benzofuro[3,2-d]pyrimidin-4-yl-1-piperazinecarbothioamide, carboplatin, 9s-(9α, 10β, 12α))-2,3,9,10,11,12-hexahydro-10-hydroxy-10-(methoxycarbonyl)-9-methyl-9,12-epoxy-1h-diindolo[1,2,3-fg:3',2',1'-kl]pyrrolo[3,4-i][1,6]benzodiazocin-1-one, oxaliplatin, etoposide, arq197, teniposide, topotecan, irinotecan, carmustine, lomustine, jnj-38877605, streptozocin, 1,3-dihydro-3-[(3,5-dimethyl-1h-pyrrol-2-yl)methylene]-2h-indol-2-one, sgx-523, aminoglutethimide, formestane, (3z)-n-(3-chlorophenyl)-3-({3,5-dimethyl-4-[(4-methylpiperazin-1-yl)carbonyl]-1h-pyrrol-2-yl}methylene)-n-methyl-2-oxo-2,3-dihydro-1h-indole-5-sulfonamide, tak-701, amix, ab1.29.1, ab1.74.1, ab1.75.1, ab2.4.4, ab2.12.1, hgf splice variants nk2 and nk4, uncleaveable hgf, cgen241, oa-5d5, isolated c-met sema domain, dn30, l2g7, pf-02341066, xl880, anastrozole, mgcd-265, genestein, rp1040, cep-a, n-[2-fluoro-4-({2-[4-(4-methylpiperazin-1-yl)piperidin-1-yl]carbonylaminopyridin-4-yl}oxy)phenyl]-n'-(4-fluorophenyl)cyclopropane-1,1-dicarboxamide(2r,3r)-tartrate, mk8033, bms777607, gsk1363089, erythropoietin, ibritumomab, levamisole, asparaginase, 2-(4-(1-(quinolin-6-ylmethyl)-1h-[1,2,3]triazolo[4,5-b]pyrazin-6-yl)-1h-pyrazol-1-yl)ethanol, procarbazine, imatinib mesylate, bevamizumab, avastin, 3-[(1r)-1-(2,6-dichloro-3-fluorophenyl)ethoxy]-5-(1-piperidin-4-ylpyrazol-4-yl)pyridin-2-amine, angiostatin and celecoxib.

10. The drug combination according to claim 6 or the pharmaceutical composition according to claim 7, wherein agent A is selected from cyclophosphamide, mechlorethamine hydrochloride, chlorambucil, 2-cyano-3-(3,4-dihydroxyphenyl)-n-(phenylmethyl)-2-propenamide, carboplalomustine, erlotinib, temozolomide, treosulfan, n-(3-(5-chloro-2-(2-methoxy-4-(4-methylpiperazin-1-yl)phenylamino)-pyrimidin-4-yloxy)phenyl)acrylamide, methotrexate, 6-thioguanine, 6-mercaptopurine, n-[-4-[(3-chloro-4-fluorophenyl)amino]-7-[3-(4-morpholinyl)-propoxy]-6-quinazolinyl]-2-propenamide, n-(3-chlorophenyl)-6,7-dimethoxy-4-quinazolinamine, fludarabine, floxuridine, gemcitabine, cytarabine, capecitabine, 4-phenethylamino-6-(yderoxyl)phenyl-7h-pyrrolo(2,3-d)pyrimidine, lapatinib, thioguanine, 7-(4-(3-ethynylphenylamino)-7-methoxyquinazolin-6-yloxy)-n-hydroxyheptanamide, mercaptopurine, paclitaxel, docetaxel, hydroxycarbamide, imatinibe, miltefosine, n-(3-(5-chloro-2-(4-(4-methylpiperazin-1-yl)phenylamino)-pyrimidin-4-yloxy)phenyl)acrylamide, cetuximab, amsacrine, 4-[(3-bromophenyl)amino]-6,7-dimethoxyquinazoline hydrochloride, trastuzumab, alemtuzumab, rituximab, n-[4-[(3-chloro-4-fluorophenyl)amino]-7-[[(3"s")-tetrahydro-3-furanyl]oxy]-6-quinazolinyl]-4(dimethylamino)-2-butenamide, gefitinib, (e)-n-(4-((3-chloro-4-fluorophenyl)amino)-3-cyano-7-ethoxyquinolin-6-yl)-4-(dimethylamino)but-2-enamide, (r)-6-(4-((4-methylpiperazin-1-yl)methyl) phenyl)-n-(1-phenylethyl)-7h-pyrrolo[2,3-d]pyrimidin-4-amine, estramustine, chlorotrianisene, diethylstilbestrol, lhrh, buserelin, goserelin, leuprorelin, triptorelin, n-(3-chloro-4-((3-fluorophenyl)methoxy)phenyl)-6-(5-(((2-(methyl sulfonyl)ethyl)amino)methyl)-2-furanyl)-4-quinazolinaminebis(4-methylbenzenesulfonate), leuprolide, goserelin acetate, [4-[[1-(3-fluorophenyl)methyl]-1h-indazol-5-ylamino]-5-methylpyrrolo[2,1-f][1,2,4]triazin-6-yl]carbamic acid (3s)-3-morpholinylmethyl ester, n-(3-(5-chloro-2-(4-(4-methylpiperazin-1-yl)phenylamino)pyrimidin-4-ylthio)phenyl)acrylamide, panitumumab.

11. The drug combination according to claim 6 or the pharmaceutical composition according to claim 7, wherein agent B is selected from (r,z)-5-((2,6-dichlorobenzyl)sulfonyl)-3-((3,5-dimethyl-4-(2-(pyrrolidin-1-ylmethyl)pyrrolidine-1-carbonyl)-1h-pyrrol-2-yl)methylene)indolin-2-one, bleomycin, epirubicin, idarubicin, amg102, dactinomycin, mitoxantrone, amg-208, plicamycin, vincristine, sch900105, vindesine, xl184, tak-701, amix, ab1.29.1, ab1.74.1, ab1.75.1, ab2.4.4, ab2.12.1, hgf splice variants nk2 and nk4, uncleaveable hgf, cgen241, oa-5d5, isolated c-met sema domain, dn30, l2g7, pf-02341066, n-((2r)-1,4-dioxan-2-ylmethyl)-n-methyl-n'-[3-(1-methyl-1h-pyrazol-4-yl)-5-oxo-5h-benzo[4,5]cyclo-hepta[1,2-b]pyridin-7-yl]sulfamide, pf-04254644, rp1040, cep-a, n-[2-fluoro-4-({2-[4-(4-methylpiperazin-1-yl)piperidin-1-yl]carbonylaminopyridin-4-yl}oxy)phenyl]-n'-(4-fluorophenyl)cyclopropane-1,1-dicarboxamide(2r,3r)-tartrate, mk8033, bms777607, gsk1363089, n-(1,3-benzodioxol-5-ylmethyl)-4-benzofuro[3,2-d]pyrimidin-4-yl-1-piperazinecarbothio amide, 9s-(9a,10β,12α))-2,3,9,10,11,12-hexahydro-10-hydroxy-10-(methoxycarbonyl)-9-methyl-9,12-epoxy-1h-diindolo[1,2,3-fg:3',2',1'-kl]pyrrolo[3,4-i][1,6]benzodiazocin-1-one, oxaliplatin, etoposide, arq197, teniposide, topotecan, irinotecan, carmustine, lomustine, jnj-38877605, streptozocin, 1,3-dihydro-3-[(3,5-dimethyl-1h-pyrrol-2-yl)methylene]-2h-indol-2-one, sgx-523, aminoglutethimide, formestane, (3z)-n-(3-chlorophenyl)-3-({3,5-dimethyl-4-[(4-methylpiperazin-1-yl)carbonyl]-1h-pyrrol-2-yl}methylene)-n-methyl-2-oxo-2,3-dihydro-1h-indole-5-sulfonamide, xl880, anastrozole, mgcd-265, genestein, erythropoietin, ibritumomab, levamisole, asparaginase, 2-(4-(1-(quinolin-6-ylmethyl)-1h-[1,2,3]triazolo[4,5-b]pyrazin-6-yl)-1h-pyrazol-1-yl)ethanol, procarbazine, imatinib mesylate, bevamizumab, avastin, 3-[(1r)-1-(2,6-dichloro-3-fluorophenyl)ethoxy]-5-(1-piperidin-4-ylpyrazol-4-yl)pyridin-2-amine, angiostatin and celecoxib.

12. Use according to any one of claims 1 - 5, wherein the proliferative disease is cancer, tumor, metastasis or a drug resistant tumor or a drug resistant cancer.
